# EUROPÄISCHE PATENTSCHRIFT

(11) **EP 1 772 099 B1**
(45) Veröffentlichungstag und Bekanntmachung des Hinweises auf die Patenterteilung: **22.06.2011**
(21) Anmeldenummer: 05021983.1
(22) Anmeldetag: 08.10.2005
(51) Int. Cl.: A61B 5/15

(54) **Stechsystem**
Piercing system
Système de perforation

(43) Veröffentlichungstag der Anmeldung: 11.04.2007
(73) Patentinhaber: Roche Diagnostics GmbH, 68305 Mannheim (DE); F. Hoffmann-La Roche AG, 4070 Basel (CH)
(72) Erfinder: List, Hans, 64754 Hesseneck-Kallbach (DE); Hoerauf, Christian, 68723 Oftersheim (DE); Deck, Frank, 67150 Niederkirchen (DE); Weiss, Thomas, 68307 Mannheim (DE); Kistner, Michael, 67071 Ludwigshafen (DE); Schmelzeisen-Redeker, Günther, 64653 Lorsch (DE); Fruhstorfer, Heinrich, 35037 Marburg (DE); Haar, Hans-Peter, 69168 Wiesloch (DE); Kraemer, Uwe, 68549 Ilvesheim (DE); Hoenes, Joachim, 64673 Zwingenberg (DE); Miltner, Karl, 67227 Frankenthal (DE)
(74) Vertreter: Mommer, Niels

(56) Entgegenhaltungen:
- EP-A- 1 243 281
- EP-A- 1 407 712
- WO-A-01/89383
- DE-A1- 10 332 283
- US-A- 5 035 704
- US-A1- 2003 018 300

## Beschreibung

Die Erfindung betrifft ein Stechgerät zum Erzeugen einer Einstichwunde für das Gewinnen einer Probe einer Körperflüssigkeit. Ein Stechgerät mit den im Oberbegriff des Anspruchs 1 angegebenen Merkmalen ist aus der US 5035704 sowie der US 2003/0018300 bekannt.

Bei der Körperflüssigkeit handelt es sich in der Regel um Blut. In manchen Fällen geht es aber auch um die Gewinnung einer Probe von interstitieller Flüssigkeit. Nachfolgend wird ohne Beschränkung der Allgemeinheit auf Blut als Beispiel, auch für andere, aus einer Einstichwunde gewinnbare Körperflüssigkeiten, Bezug genommen.

Stechsysteme bestehen in der Regel aus zur einmaligen Verwendung vorgesehenen (disposiblen) Stechelementen zum Einstechen in die Haut und einem Stechgerät mit einem Antrieb für die Stechbewegung des Stechelements. Das Stechgerät eines solchen Stechsystems hat ein Andruckteil zum Anpressen an das Körperteil, in dem eine Einstichwunde erzeugt werden soll, und ein Auslösemittel, durch dessen Betätigung ein Benutzer eine Einstichbewegung eines Stechelements auslösen kann.

Aus der DE 103 32 283 A1 ist ein Stechsystem bekannt, bei dem ein Stich erst ausgelöst werden kann, nachdem eine Sicherungseinrichtung von einer Prüfeinrichtung aus einem Sperrzustand in einen Auslösezustand versetzt wurde. Als Beispiele für die Prüfeinrichtung sind unter anderem Druck- und Temperatursensoren angegeben.

Ein ständiges Ziel in der Entwicklung von Stechsystemen besteht darin, mit möglichst geringem Schmerz eine Einstichwunde zu erzeugen, aus der sich eine brauchbare Probe, d.h. eine ausreichende Menge einer Körperflüssigkeit, gewinnen läßt. Sowohl für das Schmerzempfinden als auch für die Probengewinnung ist die Einstichtiefe von großer Bedeutung. Generell gilt, daß das Schmerzempfinden mit zunehmender Einstichtiefe ebenso wie die Flüssigkeitsmenge, die sich aus der Einstichwunde gewinnen läßt, zunehmen. An Stechgeräte besteht deshalb die Anforderung, die Einstichtiefe einerseits so gering wie möglich, andererseits so tief wie nötig zu gestalten.

In diesem Zusammenhang stellt der Anpreßdruck, mit dem das Andruckteil an ein Körperteil angepreßt wird, einen wichtigen Prüfparameter dar. Bei einem zu geringen Anpreßdruck kann es dazu kommen, daß bei einem Einstich nicht die gewünschte Einstichtiefe erreicht wird und deshalb keine brauchbare Probe gewonnen werden kann. In einem solchen Fall muß die Einstichprozedur wiederholt werden, was für einen Benutzer äußerst unangenehm ist.

Um das Risiko eines erfolglosen Einstichs zu reduzieren, sind beispielsweise aus der US 5,879,311 Stechgeräte mit einem Drucksensor bekannt, bei denen automatisch eine Einstichbewegung ausgelöst wird, sobald auf dem Andruckteil ein Druck lastet, der einen vorgegebenen Mindestdruck übersteigt.

Ein Einstichgerät mit einem eingebauten Drucksensor wird auch in der EP 1360933 A1 beschrieben. Bei diesem Stechgerät wird ein Einstich jedoch nicht automatisch durch den Drucksensor ausgelöst, sondern lediglich angezeigt, ob der auf dem Andruckteil lastende Druck in einem von drei vorgegebenen Bereichen (niedrig, normal und hoch) liegt. Das Auslösen einer Einstichbewegung geschieht durch Drücken einer Taste an dem Gerätegehäuse.

Aus der EP 1 407 712 A1 ist ebenfalls ein Stechgerät mit einem eingebauten Drucksensor bekannt. Bei diesem Gerät wird nach dem Ansetzen des Geräts an die Haut ein Unterdruck erzeugt, so daß sich das Gewebe in eine Geräteöffnung hineinwölbt. Der erzeugte Unterdruck wird mit dem Drucksensor gemessen und mit einem gespeicherten Schwellenwert verglichen. In der EP 1 407 712 A1 wird dabei sowohl die Möglichkeit angesprochen, daß eine Einstichbewegung automatisch ausgelöst wird, sobald der gemessene Unterdruck einen vorteilhaften Wert erreicht hat, als auch die Möglichkeit angesprochen, daß ein Einstich von einem Benutzer durch schließen eines Schalters ausgelöst wird und lediglich angezeigt wird, daß der gemessene Unterdruck einen vorgegebenen Schwellenwert erreicht hat.

Aufgabe der Erfindung ist es, einen Weg aufzuzeigen, wie mit möglichst großem Benutzerkomfort bei einem Stechsystem das Risiko eines erfolglosen Einstichs, der nicht zur Gewinnung einer brauchbaren Probe führt, reduziert werden kann, ohne daß durch eine unnötige große Einstichtiefe das Schmerzempfinden erhöht wird.

Diese Aufgabe wird gelöst durch ein Stechgerät mit den im Anspruch 1 angegebenen Merkmalen.

Unter der Probengewinnungswahrscheinlichkeit ist dabei die Wahrscheinlichkeit zu verstehen, mit der durch das Auslösen einer Einstichbewegung unter den vorliegenden Bedingungen, die durch den oder die ermittelten Prüfparameter charakterisiert sind, eine brauchbare Probe gewonnen wird.

Im Rahmen der vorliegenden Erfindung wurde erkannt, daß ein automatisches Auslösen der Einstichbewegung durch eine Prüfeinrichtung in Form eines Drucksensors, wie es beispielsweise bei dem aus der US 5,879,311 bekannten Stechsystem der Fall ist, vielen Benutzern aus psychologischen Gründen unangenehm ist und sogar zu einem erhöhten Schmerzempfinden führen kann. Dies wird darauf zurückgeführt, daß der Benutzer bei derartigen Stechgeräten keine Kontrolle über den genauen Zeitpunkt des Einstichs hat, der irgendwann nach dem Anpressen des Geräts erfolgt. In der Regel wartet der Benutzer deshalb einige Sekundenbruchteile oder sogar mehrere Sekunden lang auf den Einstich. Diese Wartezeit ist für viele Benutzer schwer zu ertragen, unangenehm und läßt den Einstich schmerzhafter erscheinen.

Auf der anderen Seite können bei Verwendung eines Druckmeßgeräts mit einer Druckbereichsanzeige gemäß der EP 1360933 A1 in überraschend großem Ausmaß erfolglose Einstiche (d.h. Einstiche, mit denen keine brauchbare Probe gewonnen wird) auftreten. Zum einen besteht bei einem derartigen Gerät nämlich insbesondere bei sehbehinderten Benutzern die Gefahr, daß ein Einstich ausgelöst wird, obwohl die Druckbereichsanzeige einen zu geringen Anpreßdruck anzeigt. Zum anderen kann es aber selbst dann zu einem erfolglosen Einstich kommen, wenn sich der Benutzer vor dem Betätigen der Auslösetaste vergewissert, daß der angezeigte Druck in Ordnung ist. Dies wird darauf zurückgeführt, daß manche Benutzer das Körperteil beim Betätigen der Auslösetaste reflexartig in Erwartung des Einstichs von dem Andruckteil weg bewegen oder zumindest den Anpreßdruck reduzieren.

Durch ein erfindungsgemäßes Stechsystem lassen sich die Nachteile der bekannten Geräte beheben, indem die Prüfeinrichtung mit einer Sicherungseinrichtung zusammenwirkt. Wenn der von der Prüfeinrichtung festgestellte Prüfparameter festgelegten Mindestanforderungen genügt, wird die Sicherungseinrichtung von der Prüfeinrichtung betätigt, so daß diese aus einem Sperrzustand, in dem sie das Auslösemittel sperrt, in einen Auslösezustand übergeht, in dem das Auslösemittel entsichert ist, so daß durch dessen Betätigung eine Einstichbewegung ausgelöst werden kann. Der Übergang der Sicherungseinrichtung wird dem Benutzer bevorzugt durch ein akustisches Signal mitgeteilt. Um anzuzeigen, daß sich die Sicherungseinrichtung in dem Auslösezustand befindet, kann beispielsweise ein Piepton in kurzen Zeitabständen wiederholt oder ein Signallicht eingeschaltet werden.

Auf diese Weise wird einerseits gewährleistet, daß ein Einstich nur dann erfolgt, wenn ein Benutzer das Auslösemittel betätigt. Der Benutzer hat somit die Kontrolle über den Zeitpunkt, zu dem ein Einstich erfolgt, was aus psychologischen Gründen für das Schmerzempfinden von großer Bedeutung ist. Andererseits kann verhindert werden, daß ein Einstich ausgelöst wird, wenn Prüfparameter, wie beispielsweise der Anpreßdruck, erwarten lassen, daß ein Einstich erfolglos bleiben wird. Bei einem erfindungsgemäßen Stechsystem kommt es also nur dann zu einem Einstich, wenn sowohl von der Prüfeinrichtung ein Prüfparameter festgestellt wird, der einen erfolgreichen Einstich erwarten läßt, als auch das Auslösemittel von dem Benutzer betätigt wird.

Der mit der Prüfeinrichtung eines erfindungsgemäßen Stechsystems festgestellte Prüfparameter kann beispielsweise der Anpreßdruck sein, mit dem das Andruckteil an ein Körperteil, in dem eine Einstichwunde erzeugt werden soll, angepreßt wird. Drucksensoren können beispielsweise als drucksensitives Element eine Feder enthalten. Geeignet sind ferner kapazitive Sensoren, bei denen als Maß für den Anpreßdruck eine Kapazitätsänderung auftritt, und piezoelektrische Sensoren. Alternativ oder zusätzlich kann auch die Position des Körperteils in Relation zu dem Andruckteil als Prüfparameter verwendet werden. Beispielsweise kann das Andruckteil eine relativ große Öffnung aufweisen, in die sich die Haut eines angepreßten Körperteils, beispielsweise einer Fingerbeere, abhängig von elastischen Eigenschaften der Haut je nach Patient und Handhabung unterschiedlich weit hineinwölbt. Ein weiterer möglicher Prüfparameter ist der Durchblutungszustand des an dem Andruckteil anliegenden Körperteils.

Der Durchblutungszustand kann beispielsweise mit einem optischen Sensor zur Blutsauerstoffmessung ermittelt werden. Die dafür verwendete Meßtechnik nutzt die spektrale Absorptionscharakteristik des Hämoglobins aus, beispielsweise indem ein Lichtstrahl mit definierten spektralen Intensitäten bei zwei unterschiedlichen Wellenlängen auf das anliegende Körperteil eingestrahlt wird. Das von dem Körperteil remittierte Licht wird untersucht, wobei aus der unterschiedlichen Absorption bei den beiden Wellenlängen auf den Hämoglobingehalt und damit den Durchblutungszustand des Körperteils geschlossen werden kann. Geeignete Sensoren sind beispielsweise von der Firma NONIN Medical Inc., USA, erhältlich und werden von dieser in Meßgeräten eingebaut, die unter der Marke 9500 Onyx vertrieben werden.

Ein weiterer Aspekt der Erfindung, der auch für ein Stechsystem ohne eine Sicherungseinrichtung selbständige Bedeutung haben kann, besteht darin, daß ein Benutzer beim Ausrichten eines Andruckteils auf das Körperteil, in dem eine Einstichwunde erzeugt werden soll, durch Signale unterstützt wird, die dem Benutzer eine Information über die Orientierung des Andruckteils zu dem Körperteil vermitteln. Insbesondere für ältere Patienten ist es mühsam, den richtigen Umgang mit einem Stechsystem zu erlernen. Vielen Patienten bereitet dabei das korrekte Ausrichten des Körperteils, insbesondere hinsichtlich Position und Anpreßdruck auf den Andruckteil, Probleme.

Durch eine Prüfeinrichtung kann dem Benutzer diesbezüglich eine Rückmeldung gegeben werden, so daß die korrekte Handhabung des Stechsystems leichter und schneller erlernt werden kann. Bevorzugt ermöglicht die Prüfeinrichtung eine visuelle Kontrolle des an dem Körperteil anliegenden Andruckteils und/oder des an dem Andruckteil anliegenden Körperteils. Beispielsweise kann hierfür in das Stechgerät eine Kamera integriert sein, so daß ein entsprechendes Bild aufgenommen und mittels einer Anzeigeeinrichtung angezeigt werden kann. Anstelle einer Kamera kann eine visuelle Kontrolle auch mit passiven optischen Elementen, beispielsweise Spiegeln und/oder Linsen, ermöglicht werden.

Allgemein gesagt kann die Prüfeinrichtung den Benutzer beim Ausrichten des Andruckteils auf das Körperteil auch durch optische, akustische oder sonstige Signale unterstützen. Beispielsweise kann das Andruckteil so ausgebildet sein, daß es sich für den Benutzer fühlbar verformt, sobald ein Druck aufgebracht wird, der einen vorgegebenen Mindestanpreßdruck übersteigt. Auf diese Weise kann ein tastbares Signal erzeugt werden.

Weitere Einzelheiten und Vorteile der Erfindung werden anhand von Ausführungsbeispielen unter Bezugnahme auf die beigefügten Zeichnungen erläutert. Gleiche oder einander entsprechende Bauteile sind dabei mit übereinstimmenden Bezugszeichen gekennzeichnet.

Die im folgenden beschriebenen Merkmale können einzeln oder in Kombination verwendet werden, um bevorzugte Ausgestaltungen der Erfindung zu schaffen. Es zeigen:
- Fig. 1: ein Ausführungsbeispiel eines erfindungsgemäßen Stechsystems in einer teilweise geschnittenen Seitenansicht;
- Fig. 2: das in Figur 1 dargestellte Ausführungsbeispiel in einem entsicherten Zustand;
- Fig. 3: ein weiteres Ausführungsbeispiel eines erfindungsgemäßen Stechsystems in einer Seitenansicht;
- Fig. 4: das in Figur 3 dargestellte Ausführungsbeispiel mit durch Druck verformtem Andruckteil;
- Fig. 5: das Andruckteil eines weiteren Ausführungsbeispiels eines erfindungsgemäßen Stechsystems im Querschnitt;
- Fig. 6: ein weiteres Ausführungsbeispiel eines Andruckteils eines erfindungsgemäßes Stechsystems in einer Seitenansicht;
- Fig. 7: das in Figur 6 dargestellte Andruckteil mit einem angepreßten Finger;
- Fig. 8: eine Prinzipskizze zur Messung des Durchblutungszustands eines an dem Andruckteil anliegenden Körperteils;
- Fig. 9: ein weiteres Ausführungsbeispiel eines erfindungsgemäßen Stechsystems in einer teilweise geschnittenen Seitenansicht;
- Fig. 10: das in Fig. 9 dargestellte Ausführungsbeispiel unter Einwirkung eines Anpreßdrucks;
- Fig. 11: ein weiteres Ausführungsbeispiel des erfindungsgemäßen Stechsystems in einer teilweise geschnittenen Seitenansicht; und
- Fig. 12: das in Fig. 11 dargestellte Ausführungsbeispiel unter Einwirkung eines Anpreßdrucks.

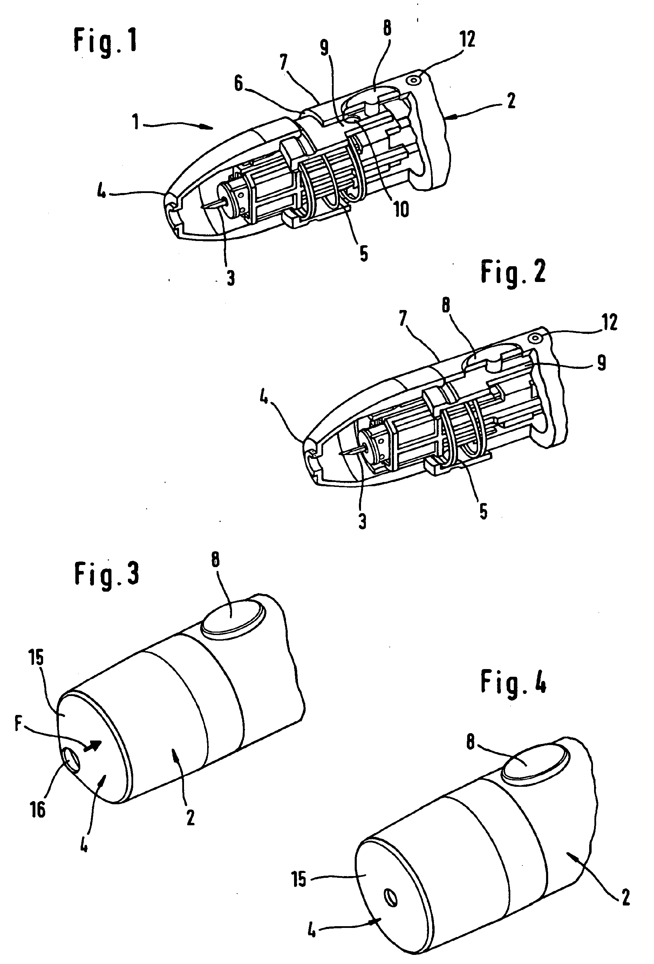

Das in Figur 1 dargestellte Stechsystem 1 umfaßt ein Stechgerät 2 und zur einmaligen Verwendung vorgesehene Stechelemente 3, die in das Stechgerät 2 eingesetzt werden. Zum Erzeugen einer Einstichwunde wird das Stechgerät 2 mit seinem Andruckteil 4 an eine Hautoberfläche eines Benutzers angepreßt. Überschreitet der dabei ausgeübte Anpreßdruck einen durch die Stärke einer Feder 5 festgelegten Mindestdruck, so wird das Andruckteil 4 gegen eine Kante 6 des Gerätegehäuses 7 geschoben.

Das dargestellte Stechgerät 2 weist ein Auslösemittel 8 auf, durch dessen Betätigung ein Benutzer nach dem Anpressen des Andruckteils 4 eine Einstichbewegung auslösen kann. Bei dem dargestellten Ausführungsbeispiel ist das Auslösemittel 8 als Knopf ausgebildet. Um zu gewährleisten, daß nur dann ein Einstich ausgelöst wird, wenn dieser voraussichtlich zur Gewinnung einer brauchbaren Probe führt, ist das Stechgerät 2 mit einer Sicherungseinrichtung 9 ausgestattet, die in dem in Figur 1 dargestellten Sperrzustand das Auslösemittel 8 sperrt, so daß keine Einstichbewegung ausgelöst werden kann, und in einem in Figur 2 dargestellten Auslösezustand das Auslösemittel 8 freigibt, so daß durch dessen Betätigung eine Einstichbewegung ausgelöst werden kann.

Bei dem dargestellten Ausführungsbeispiel ist die Sicherungseinrichtung 9 als eine an dem Andruckteil 4 angebrachte Sperrzunge ausgebildet. In dem in Figur 1 dargestellten Sperrzustand wird das als Auslöseknopf ausgebildete Auslösemittel 8 mechanisch von der Sperrzunge 9 blockiert. Die Sperrzunge 9 hat eine Aussparung 10. Wird das Andruckteil 4 gegen die Kante 6 des Gerätegehäuses 7 geschoben, so wird auch die Sperrzunge 9 gegenüber dem Auslöseknopf 8 verschoben, bis die Aussparung 10 mit dem Auslöseknopf 8 fluchtet und so der in Figur 2 dargestellte Auslösezustand der Sicherungseinrichtung 9 erreicht wird. In diesem Zustand ist der Auslöseknopf 8 nicht mehr blockiert, so daß durch Drücken des Auslöseknopfs 8 eine Einstichbewegung ausgelöst werden kann.

Alternativ kann die Sicherungseinrichtung 9 auch als Schalter eines Auslösestromkreises ausgebildet sein. Beispielsweise kann eine Einstichbewegung durch Schließen des Auslösestromkreises ausgelöst werden, wobei ein erster Schalter, der von der Sicherungseinrichtung gebildet und bei Kontakt des Andruckteils 4 mit der Gehäusekante geschlossen wird, und ein zweiter Schalter, der durch Betätigen des Auslösemittels 8 geschlossen wird, in dem Stromkreis angeordnet sind.

Das dargestellte Stechsystem hat den wichtigen Vorteil, daß der Benutzer durch Betätigen des Auslösemittels 8 nur dann einen Einstich auslösen kann, wenn der Anpreßdruck so hoch ist, daß eine Einstichbewegung voraussichtlich eine brauchbare Körperflüssigkeitsprobe liefert. Erfolglose Einstiche werden durch eine Prüfeinrichtung, die bei dem dargestellten Ausführungsbeispiel einen Drucksensor in Form einer Feder 5 umfaßt, vermieden.

Der Übergang der Sicherungseinrichtung 9 aus dem Sperrzustand in den Auslösezustand wird einem Benutzer durch ein geeignetes Signal angezeigt. Zu diesem Zweck dient eine Signaleinrichtung 12, beispielsweise eine Leuchtdiode, mit der durch ein Leuchtsignal angezeigt wird, daß sich die Sicherungseinrichtung 9 in dem Auslösezustand befindet und folglich durch Betätigen des Auslösemittels 8 ein Einstich ausgelöst werden kann. Alternativ oder zusätzlich kann die Signaleinrichtung 12 auch ein akustisches Signal, beispielsweise einen Piepton, erzeugen, um den Übergang der Sicherungseinrichtung 9 in den Auslösezustand anzuzeigen.

In den Figuren 3 und 4 ist ein weiteres Ausführungsbeispiel eines Stechsystems dargestellt, bei dem die Prüfeinrichtung des Stechgeräts 2 einen Drucksensor in Form eines Schnappelements 15 umfaßt, das bei Einwirkung eines kritischen Mindestdrucks aus einer ersten Konfiguration, die in Figur 3 dargestellt ist, in eine zweite Konfiguration, die in Figur 4 dargestellt ist, schnappt. Ein derartiges Schnappelement stellt einen besonders vorteilhaften Drucksensor dar, der auch für Stechsysteme ohne eine Sicherungseinrichtung vorteilhaft zu verwenden ist und deshalb einen Aspekt der Erfindung darstellt, der auch selbständige Bedeutung hat. Das Schnappelement 15 kann beispielsweise ein Metallblech sein, das beim Umschnappen ein hörbares Knackgeräusch erzeugt. In der in Figur 3 dargestellten ersten Konfiguration hat das Schnappelement 15 eine kegelstumpfähnliche Form mit einer Öffnung 16, in die sich eine Hautoberfläche eines angelegten Körperteils hineinwölben kann.

Sobald eine Mindestandruckkraft von beispielsweise 10 Newton in Pfeilrichtung F auf das Schnappelement 15 ausgeübt wird, schnappt es hör- und fühlbar in die in Figur 4 dargestellte zweite Konfiguration. In dieser Konfiguration bleibt es, solange ein Haltedruck ausgeübt wird, der etwas geringer als der zum Umschnappen in die zweite Konfiguration erforderliche kritische Mindestdruck ist und beispielsweise 7 Newton beträgt. Wird dieser Haltedruck unterschritten, so schnappt das Schnappelement 15 federnd in seine erste Konfiguration zurück.

Der kritische Mindestdruck, bei dessen Überschreiten das Schnappelement 15 aus der ersten Konfiguration in die zweite Konfiguration schnappt, und der Haltedruck, bei dessen Unterschreiten das Schnappelement 15 aus der zweiten Konfiguration zurück in die erste Konfiguration schnappt, unterscheiden sich bevorzugt um wenige Newton, beispielsweise 1 bis 3 Newton. Wichtig ist, daß der Haltedruck so gewählt wird, daß bei Anpressen des Andruckteils 4 mit dem Haltedruck an ein Körperteil Bedingungen vorliegen, die mit möglichst hoher Wahrscheinlichkeit erwarten lassen, daß das Auslösen einer Einstichbewegung eine Einstichwunde erzeugt, mit der eine brauchbare Probe gewonnen wird.

Das beschriebene Schnappelement 15 stellt ein Beispiel für eine Signaleinrichtung dar, die in die Prüfeinrichtung integriert ist, da durch das Umschnappen sowohl festgestellt wird, daß der als Prüfparameter erfaßte Anpreßdruck festgelegten Mindestanforderungen genügt, als auch ein akustisches Signal erzeugt wird.

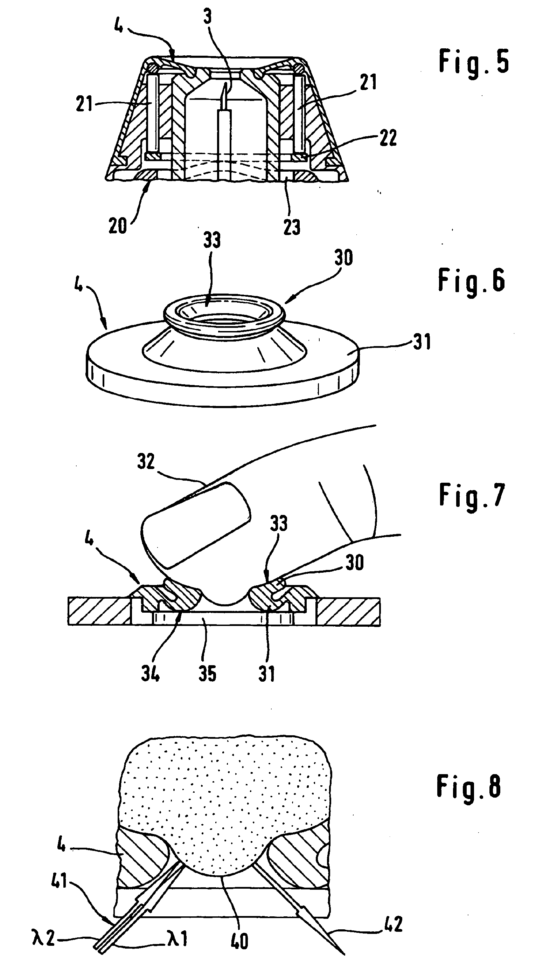

In Figur 5 sind das Andruckteil 4 und die Prüfeinrichtung 20 eines weiteren Ausführungsbeispiels in einer Querschnittsansicht dargestellt. Die Prüfeinrichtung 20 dieses Ausführungsbeispiels hat den Vorteil, daß zusätzlich zu dem Anpreßdruck auch die Ausrichtung des Andruckteils 4 relativ zu einem anliegenden Körperteil geprüft werden kann. Die Prüfeinrichtung 20 umfaßt zu diesem Zweck mehrere, bei dem dargestellten Ausführungsbeispiel zwei, Sensorstifte 21, die in ihrer Längsrichtung beweglich angeordnet sind. Wird auf das Andruckteil 4 Druck ausgeübt, so werden die Sensorstifte 21 gegen ein Kippelelement 22 geschoben, das bei dem dargestellten Ausführungsbeispiel als Ring ausgebildet ist. Das Kippelement 22 ist auf einem Sensorelement 23 so gelagert, daß es kippt, wenn die Sensorstifte 21 um unterschiedliche Strecken zurückgeschoben wurden. Nur wenn die Sensorstifte 21 um gleiche Strecken zurückgeschoben wurden, wird der auf den Sensorstiften 21 lastende Anpreßdruck auf das Sensorelement 23 übertragen, so daß dieses gegen eine Rückstellfeder (nicht dargestellt) zurückgeschoben wird.

Wird bei der beschriebenen Prüfeinrichtung das Sensorelement 23 um eine kritische Strecke zurückgeschoben, die durch die Härte der Rückstellfeder vorgegeben werden kann, so bedeutet dies, daß der auf den Sensorstiften 21 lastende Druck im wesentlichen gleichmäßig verteilt ist und einen kritischen Mindestanpreßdruck übersteigt.

Die anhand von Figur 5 beschriebene Prüfeinrichtung umfaßt also einen Drucksensor, mit dem feststellbar ist, ob das Andruckteil 4 auf eine Hautoberfläche einen im voraus festgelegten Druck ausübt, und ob gleichzeitig das Andruckteil 4 so ausgerichtet ist, daß eine Einstichbewegung eines Stechelements 3 im wesentlichen senkrecht zu der Hautoberfläche erfolgen würde. Als Prüfparameter kann auf diese Weise unter anderem die Orientierung des Andruckteils 4 zu dem anliegenden Körperteil ermittelt werden. Wird die beschriebene Prüfeinrichtung 20 an eine Anzeigeeinrichtung angeschlossen, so kann ein Benutzer bei dem Ausrichten des Andruckteils auf das Körperteil, in dem eine Einstichwunde erzeugt werden soll, durch Signale unterstützt werden, die dem Benutzer eine Information über die Orientierung des Andruckteils 4 zu dem Körperteil vermitteln.

Der anhand von Figur 5 beschriebene Drucksensor, mit dem eine Positionserkennung möglich ist, ist in anderem Zusammenhang, nämlich für ein Gerät zur nadellosen Injektion von Medikamenten, bekannt und in der EP 1243281 B1 beschrieben, deren diesbezüglicher Inhalt durch Bezugnahme zum Gegenstand der vorliegenden Anmeldung gemacht wird.

In den Figuren 6 und 7 ist ein weiteres Ausführungsbeispiel eines Andruckteils 4 eines Stechgeräts dargestellt. Das dargestellte Andruckteil 4 ist als Kompressionskonus ausgebildet, mit dem die Probengewinnung durch eine erhöhte Durchblutung des Gewebes an der Einstichstelle unterstützt wird. Als Material für den dargestellte Kompressionskonus eignen sich beispielsweise gummielastische Kunststoffe.

Das Andruckteil 4 hat einen trichterförmigen Andruckring 30, der auf einem kegelstumpfförmigen Trägerbereich 31 sitzt. Beim Anlegen eines Körperteils 32 schmiegen sich die Innenflächen des Andruckrings 30 an die Haut an und bewirken eine erhöhte Durchblutung des sich in das Andruckteil 4 hinein wölbenden Gewebes. Übersteigt der auf dem Andruckteil 4 lastende Druck einen vorgegebenen Mindestdruck, so geht das Andruckteil 4 aus der in Figur 6 dargestellten ersten Konfiguration in die in Figur 7 dargestellte zweite Konfiguration über, indem die Wände des Trägerbereichs 31 für den Benutzer spürbar nachgeben.

Das Umklappen des Andruckteils 4 aus dem in Figur 6 dargestellten ersten Zustand in den in Figur 7 dargestellten zweiten Zustand kann beispielsweise mit einem elektrischen Kontakt detektiert werden, der geschlossen wird, wenn eine geeignete Stelle 34 der Innenseite des Trägerbereichs 31 einen Basisring 35 berührt. Alternativ kann die Verformung des Andruckteils 4 beispielsweise auch mittels eines optischen Sensors detektiert werden. Weitere Einzelheiten und Vorteile des beschriebenen Kompressionskonus sind in der WO 01/89383 A2 beschrieben, die diesbezüglich durch Bezugnahme zum Gegenstand der vorliegenden Anmeldung gemacht wird.

Bei dem anhand der Figuren 6 und 7 beschriebenen Ausführungsbeispiel wird als Prüfparameter also ebenfalls festgestellt, ob auf dem Andruckteil 4 ein Druck lastet, der einen vorgegebenen Mindestanpreßdruck übersteigt. Dieser Prüfparameter ist für die Probengewinnungswahrscheinlichkeit relevant, mit der durch das Auslösen einer Einstichbewegung unter den vorliegenden Bedingungen, die durch den Prüfparameter charakterisiert sind, eine brauchbare Probe gewonnen wird. Wird der vorgegebene Mindestanpreßdruck überschritten, kann davon ausgegangen werden, daß mit einer Einstichbewegung eine Einstichwunde erzeugt werden kann, die eine brauchbare Probe liefert.

Alternativ oder zusätzlich kann die Prüfeinrichtung ferner einen Sensor umfassen, mit dem als Prüfparameter der Durchblutungszustand eines an dem Andruckteil 4 anliegenden Körperteils ermittelbar ist.

Der Durchblutungszustand ist von großer Bedeutung für die Frage, ob mittels einer Einstichbewegung mit eingestellten Einstichtiefe eine brauchbare Probe gewonnen werden kann. Wird beispielsweise eine Fingerbeere gegen eine Öffnung des Andruckteils gepreßt, so wölbt sich die Hautoberfläche in die Öffnung des Andruckteils 4 hinein. Dies ist schematisch in Figur 8 dargestellt. Der Durchblutungszustand des sich in die Öffnung hineinwölbenden Gewebes 40 hängt u.a. von der Ausrichtung des Andruckteils 4 zu dem Finger und dem Anpreßdruck ab.

Die Messung des Durchblutungszustandes basiert auf der spektralen Absorptionscharakteristik des Hämoglobins. Bevorzugt wird ein Zwei-Wellenlängen-Meßverfahren angewandt, da sich entsprechende Sensoren sehr kompakt fertigen lassen. Bei einem Zwei-Wellenlängen-Meßverfahren wird das Gewebe an der Einstichstelle mit Primärlicht 41 bestrahlt und die spektrale Intensität des remittierten Lichts 42 bei zwei Wellenlängen λ1, λ2 gemessen.

Diese beiden Wellenlängen λ1, λ2 sind so gewählt, daß Hämoglobin bei einer dieser beiden Wellenlängen eine hohe Absorption und bei der anderen Wellenlänge ebenso wie das Gewebe 40 eine möglichst geringe Absorption hat. Aus dem Verhältnis der Lichtintensitäten des remittierten Lichts 42 bei den beiden Wellenlängen λ1, λ2 kann auf den Hämoglobingehalt des Gewebes 40 und damit auf den Durchblutungszustand des Körperteils geschlossen werden. Wichtig dabei ist lediglich, daß das Verhältnis der spektralen Lichtintensitäten I(λ1), I(λ2) des Primärlichts 41 bei den beiden Wellenlängen λ1, λ2 bekannt ist. Falls erforderlich kann dieses Verhältnis durch eine separate Messung von Primärlicht 41 ermittelt werden. Bevorzugt wird jedoch eine Primärlichtquelle verwendet, bei der das Verhältnis der beiden Lichtintensitäten I(λ1): I(λ2) konstant ist.

In Figur 8 ist das Prinzip des beschriebenen Zwei-Wellenlängen-Meßverfahrens schematisch dargestellt. In dem Primärlicht 41 sind die beiden Wellenlängen durch zwei Pfeile λ1, λ2 dargestellt, die zu dem zu untersuchenden Gewebe 40 hindeuten. Licht der Wellenlänge λ1 wird von Hämoglobin stark absorbiert, so daß die spektrale Intensität I(λ1) bei der Wellenlänge λ1 in dem remittierten Licht 42 im Verhältnis zu der spektralen Intensität I(λ2) bei der Wellenlänge λ2 sehr klein ist.

Bei dem beschriebenen Verfahren kann als Prüfparameter das Verhältnis der Lichtintensitäten I(λ1): I(λ2) des remittierten Lichts 42 bei den beiden Wellenlängen λ1, λ2 verwendet werden. Durch Vergleich mit einem kritischen Schwellenwert kann festgestellt werden, ob bei den durch den so gewonnenen Prüfparameter charakterisierten Bedingungen ein Einstich voraussichtlich zur Gewinnung einer brauchbaren Probe führen würde.

Einen besonders hohen Benutzerkomfort bieten Stechsysteme, mit denen nicht nur eine Einstichwunde erzeugt wird, sondern die zusätzlich auch eine Probeaufnahmeeinheit umfassen, mit der nach einem Einstich während einer Sammelphase eine Körperflüssigkeitsprobe aufgenommen wird. Bevorzugt ist die Probenaufnahmeeinheit in das Stechelement integriert, beispielsweise indem das Stechelement einen Kapillarkanal aufweist, in den Körperflüssigkeit während der Sammelphase eindringt. Bei derartigen Stechsystemen macht die Probenaufnahme für den Benutzer keine zusätzlichen Handhabungsschritte erforderlich. Dies ist insbesondere für Benutzer, deren motorische Beweglichkeit durch Alter oder Krankheit eingeschränkt ist, ein wichtiger Vorteil.

Bevorzugt enthält das Stechgerät 2 eine Meßeinheit zum Untersuchen einer gewonnenen Körperflüssigkeitsprobe auf einen medizinisch bedeutsamen Analyten, beispielsweise Glucose. Damit für eine zuverlässige Analyse eine brauchbare Probe gewonnen werden kann, ist es wichtig, daß nicht nur bei dem Einstich, sondern auch einer darauffolgenden Sammelphase standardisierte Bedingungen, beispielsweise hinsichtlich Anpreßdruck und Positionierung des Körperteils an dem Andruckteil 4, erhalten bleiben. Bevorzugt bleibt deshalb die Prüfeinrichtung bis zum Ende der Sammelphase aktiv und zeigt einen für eine Probenaufnahme nachteilige Änderung des mindestens einen Prüfparameters durch ein Warnsignal an.

Bei einer mehr als eine Sekunde andauernden Sammelphase enthält ein Benutzer durch ein derartiges Warnsignal die Möglichkeit auf eine nachteilige Änderung des Prüfparameters zu reagieren, so daß (falls erforderlich) ein nachlassender Anpreßdruck wieder erhöht oder eine fehlerhafte Positionierung des Körperteils korrigiert werden kann.

Bevorzugt ist die Prüfeinrichtung an die Meßeinheit angeschlossen, so daß bei einer während der Sammelphase auftretenden kritischen Änderung eines Prüfparameters eine Analyse der gewonnenen Probe verhindert wird. Auf diese Weise lassen sich fehlerhafte Meßergebnisse, die beispielsweise einen Benutzer zu einer falschen Insulindosierung verleiten könnten, vermeiden. Ein weiterer Vorteil dieser Maßnahme besteht darin, daß bei Meßeinheiten, die zur Untersuchung einer Körperflüssigkeitsprobe ein von dem Stechelement separates Testelement verwenden, der unnötige Gebrauch eines solchen Testelements eingespart werden kann.

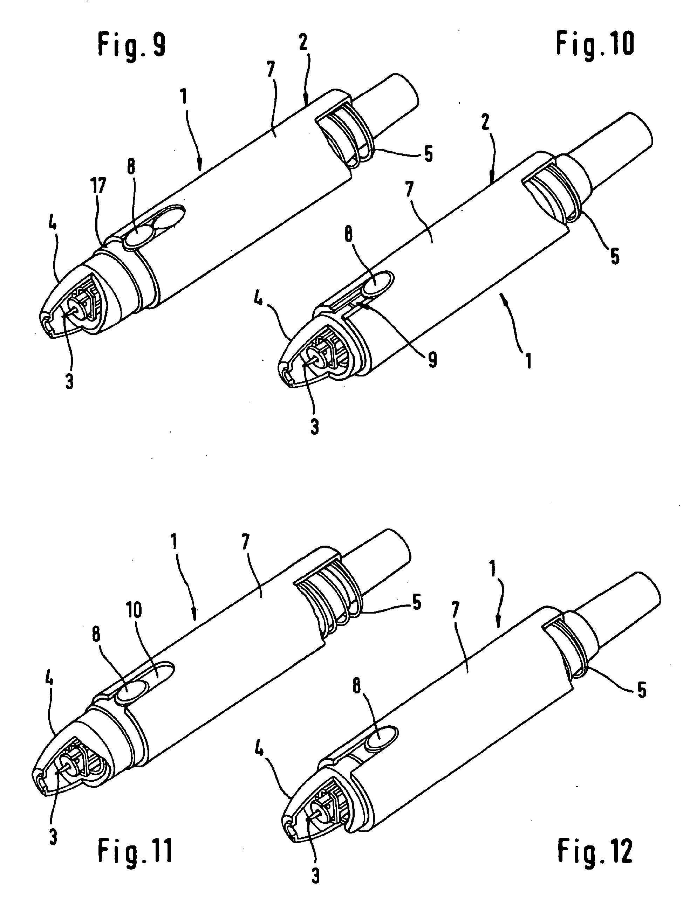

Das in Fig. 9 dargestellte Stechsystem 1 entspricht in seinem Aufbau weitgehend dem anhand von Fig. 1 beschriebenen Stechsystem. Es umfaßt ein Stechgerät 2 und zur einmaligen Verwendung vorgesehene Stechelemente 3, die in das Stechgerät 2 eingesetzt werden. Zum Erzeugen einer Einstichwunde wird das Stechgerät 2 mit seinem Andruckteil 4 an eine Hautoberfläche eines Benutzers angepreßt. Das Andruckteil 4 ist relativ zu einem Gehäuseteil 7 gegen die Federkraft einer Feder 5 derart verschieblich, daß das Andruckteil 4 bei Aufbringen eines vorgegebenen Mindestdrucks gegenüber dem Gehäuseteil 7 aus der in Figur 9 dargestellten Anfangsposition in eine Endposition gelangt, die in Fig. 10 dargestellt ist.

Das Federelement 5 stellt also den Drucksensor einer Prüfeinrichtung dar, mit dem als Prüfparameter feststellbar ist, ob auf dem Andruckteil 4 ein Druck lastet, der einen vorgegebenen Mindestanpreßdruck übersteigt.

Gemäß der Erfindung ist das Andruckteil 4 starr mit einem Stechelementantrieb (nicht dargestellt) gekoppelt, so daß eine eingestellte Einstichtiefe nicht von einer Verschiebung des Andruckteils 4 gegenüber dem Gehäuseteil 7 beeinflußt wird. Das Gehäuseteil 7 ist zu diesem Zweck als eine Hülse ausgebildet, die einen inneren Geräteteil umgibt, zu dem das Andruckteil 4 und der Stechelementantrieb gehören. Die zwischen dem Andruckteil 4 und dem Gehäuseteil 7 wirkende Feder 5 ist als Schraubenfeder ausgebildet, die sich mit einem Ende an einer Schulter des Gehäuseteils 7 abstützt und mit ihrem anderen Ende an dem Andruckteil 4 befestigt ist.

Das dargestellte Stechgerät 2 weist ein Auslösemittel 8 auf, durch dessen Betätigung ein Benutzer nach dem Anpressen des Andruckteils 4 eine Einstichbewegung auslösen kann. Das Auslösemittel 8 ist als Knopf ausgebildet, der nur dann gedrückt werden kann, wenn er mit einer passenden Aussparung 10 des Gehäuseteils 7 fluchtet. Dies ist der Fall, wenn das Gehäuseteil 7 gegenüber dem Andruckteil 4 in die in Fig. 10 dargestellte Endposition gelangt ist.

Bei dem in den Figuren 9 und 10 dargestellten Ausführungsbeispiel wird eine Sicherungseinrichtung 9 von einem Schlitz gebildet, der in die Aussparung 10 mündet und in dem der Auslöseknopf beim Verschieben des Andruckteils in die in Figur 10 gezeigte Endposition gleitet. Der Schlitz 9 ist so schmal, daß ein Einstich nur dann ausgelöst werden kann, wenn das Andruckteil 4 mit einem ausreichenden Mindestanpreßdruck an ein Körperteil angepreßt wird.

Durch das Verschieben des Gehäuseteils 7 gegenüber dem Andruckteil 4 kann einem Benutzer mit einfachen Mitteln, nämlich auf mechanische Weise, signalisiert werden, daß das Stechgerät 2 mit einem ausreichenden Anpreßdruck an ein Körperteil angepreßt wird. Auf aufwendige elektronische Anzeigeeinrichtungen kann deshalb verzichtet werden. Um das Erreichen der in Fig. 10 dargestellten Endposition für ein Benutzer noch deutlicher zu machen, weist das Andruckteil 4 eine sichtbare Markierung 17, bevorzugt als Farbmarkierung in Form eines Farbrings, auf, die vor dem Anpressen für einen Benutzer sichtbar ist und in der in Fig. 10 dargestellten Endposition des Andruckteils 4 verdeckt ist.

Das Unterscheiden der Anfangsposition von der Endposition wird mittels der Markierung 17 erleichtert. Anstelle einer Farbmarkierung kann beispielsweise auch eine geometrische Markierung, beispielsweise ein Kreis oder Quadrat, verwendet werden.

Die Markierung 17 kann entweder wie bei dem dargestellten Ausführungsbeispiel auf dem Andruckteil angebracht werden, und in der Endposition von dem Gehäuseteil 7 verdeckt werden. Möglich ist es aber auch, das Gehäuseteil 7 mit einem Fenster zu versehen, mit dem die Markierung 17 zusammenwirkt. Je nach Ausgestaltung des Geräts ist die Markierung nur dann zu sehen, wenn sich das Andruckteil 4 in der Endposition befindet, oder alternativ nur dann, wenn sich das Andruckteil 4 in der Anfangsposition befindet. Möglich ist es auch, die Markierung auf dem Gehäuseteil 7 anzubringen und das Andruckteil 4 entsprechend abzuwandeln, so daß eine auf dem Gehäuseteil 7 angebrachte Markierung entweder in der Endposition oder in der Anfangsposition von dem Andruckteil 4 verdeckt wird.

Der Vorteil einer einfachen Signalisierung günstiger Andruckbedingungen kann auch bei einem Stechgerät 2 ohne eine Sicherungseinrichtung 9 genutzt werden. In den Fig. 11 und 12 ist ein Ausführungsbeispiel eines Stechsystems 1 dargestellt, daß sich von dem anhand der Figuren 9 und 10 beschriebenen Stechsystem lediglich dadurch unterscheidet, daß keine Sicherungseinrichtung 9 vorhanden ist. Bei dem in den Figuren 11 und 12 dargestellten Ausführungsbeispiel ist nämlich die Ausnehmung 10 des Gehäuseteils 7 als ein so breiter Schlitz ausgebildet, daß das Auslösemittel 8 in jeder beliebigen Position des Andruckteils 4 gegenüber dem Gehäuseteil 7 betätigt und ein Einstich ausgelöst werden kann.

## Patentansprüche

1. Stechgerät zum Erzeugen einer Einstichwunde für das Gewinnen einer Probe einer Körperflüssigkeit, umfassend
ein Andruckteil (4) zum Anpressen an ein Körperteil (32), in dem eine Einstichwunde erzeugt werden soll, ein Auslösemittel (8), durch dessen Betätigung ein Benutzer nach dem Anpressen des Andruckteils (4) eine Einstichbewegung eines Stechelements (3) auslösen kann,
eine Prüfeinrichtung (5,15,20) zum Ermitteln mindestens eines Prüfparameters, von dem eine Probengewinnungswahrscheinlichkeit abhängt, wobei die Prüfeinrichtung (5) eine Drucksensor umfaßt, mit dem als Prüfparameter feststellbar ist, ob auf dem Andruckteil (4) ein Druck lastet, der einen vorgegebenen Mindestwert übersteigt, wobei
der Drucksensor (5) ein Federelement umfaßt, gegen dessen Federkraft das Andruckteil (4) relativ zu einem Gehäuseteil (7) derart verschieblich ist, daß das Andruckteil (4) bei Aufbringen eines Drucks, der den vorgegebenen Mindestwert übersteigt, relativ zu dem Gehäuseteil (7) in eine Endposition gelangt, **dadurch gekennzeichnet, daß**
das Andruckteil (4) starr mit einem Stechelementantrieb verbunden ist.

2. Stechgerät nach Anspruch 1, **dadurch gekennzeichnet, daß** beim Verschieben des Andruckteils (4) in die Endposition eine sichtbare Markierung, vorzugsweise eine Farbmarkierung, verdeckt oder freigegeben wird.

3. Stechgerät nach einem der vorstehenden Ansprüche, **dadurch gekennzeichnet, daß** die sichtbare Markierung (17) auf dem Andruckteil (4) angeordnet ist und das Gehäuseteil (7) ein Sichtfenster aufweist, durch das die Markierung (17) sichtbar ist, wenn sich das Andruckteil (4) in der Endposition befindet.

4. Stechgerät nach einem der vorstehenden Ansprüche, **gekennzeichnet durch**
eine Sicherungseinrichtung (9), die in einem Sperrzustand das Auslösemittel (8) sperrt, so daß keine Einstichbewegung ausgelöst werden kann, und in einem Auslösezustand das Auslösemittel (8) freigibt, so daß durch dessen Betätigung eine Einstichbewegung ausgelöst werden kann, wobei
die Sicherungseinrichtung (9) von der Prüfeinrichtung (5,15,20) aus dem Sperrzustand in den Auslösezustand versetzt wird, wenn der von der Prüfeinrichtung (5,15,20) festgestellte Prüfparameter festgelegten Mindestanforderungen genügt, und
eine Signaleinrichtung (12) zum Signalisieren eines Übergangs der Sicherungseinrichtung (9) von dem Sperrzustand in den Auslösezustand und/oder zum Signalisieren, daß sich die Sicherungseinrichtung in dem Auslösezustand befindet.

5. Stechgerät nach Anspruch 4, **dadurch gekennzeichnet, daß** die Prüfeinrichtung (5,15,20) einen Sensor umfaßt, mit dem als Prüfparameter der Durchblutungszustand eines an dem Andruckteil (4) anliegenden Körperteils ermittelbar ist.

6. Stechgerät nach einem der vorstehenden Ansprüche, **dadurch gekennzeichnet, daß** der Drucksensor (5,15) ein Schnappelement umfaßt, das bei Einwirkung eines kritischen Mindestdrucks aus einer ersten Konfiguration in eine zweite Konfiguration schnappt.

7. Stechgerät nach Anspruch 6, **dadurch gekennzeichnet, daß** das Schnappelement (15) ein Metallblech ist.

8. Stechgerät nach einem der Ansprüche 4 bis 7, **dadurch gekennzeichnet, daß** die Prüfeinrichtung (5,15,20) einen Benutzer bei dem Ausrichten des Andruckteils (4) auf das Körperteil (32), in dem eine Einstichwunde erzeugt werden soll, durch Signale unterstützt, die dem Benutzer eine Information über die Orientierung des Andruckteils (4) zu dem Körperteil (32) vermitteln.

9. Stechgerät nach einem der Ansprüche 4 bis 8, **dadurch gekennzeichnet, daß** die Prüfeinrichtung (5,15,20) eine visuelle Kontrolle des an dem Körperteil (32) anliegenden Andruckteils (4) und/oder des an dem Andruckteil (4) anliegenden Körperteils (32) ermöglicht.

10. Stechgerät nach einem der Ansprüche 4 bis 9, **dadurch gekennzeichnet, daß** es eine Probenaufnahmeeinheit umfaßt, mit der nach einem Einstich während einer Sammelphase eine Körperflüssigkeitsprobe aufgenommen wird, wobei die Prüfeinrichtung (5,15,20) bis zum Ende der Sammelphase aktiv bleibt und eine für eine Probenaufnahme nachteilige Änderung des mindestens einen Prüfparameters durch ein Warnsignal anzeigt.

11. Stechgerät nach Anspruch 10, **dadurch gekennzeichnet, daß** es eine Meßeinheit zum Untersuchen einer gewonnenen Körperflüssigkeitsprobe auf einen medizinisch bedeutsamen Analyten hat, wobei die Prüfeinrichtung (5,15,20) an die Meßeinheit angeschlossen ist, so daß bei einer während der Sammelphase auftretenden kritischen Änderung eines Prüfparameters eine Analyse der gewonnenen Probe verhindert wird.

## Claims

1. Puncturing device, in particular for a puncturing system according to any one of the preceding claims, for generating a puncturing wound for obtaining a sample of a body fluid, comprising
a press-on part (4) to be pressed onto a body part (32), in which a puncturing wound is to be generated;
a triggering means (8), by the actuation of which a user can trigger a puncturing motion of a puncturing element (3) after the press-on part (4) is pressed on;
a testing facility (5,15,20) for determining at least one test parameter on which a sample-obtaining probability depends, whereby the testing facility (5) comprises a pressure sensor that can be used to determine, as test parameter, whether or not a pressure that exceeds a predetermined minimal pressure bears on the press-on part (4), wherein the pressure sensor (5) comprises a spring element, against the spring force of which the press-on part (4) can be shifted with respect to a housing part (7) such that the press-on-part (4) reaches an end position with respect to the housing part (7) when the minimal pressure is applied,
**characterized in that** the press-on part (4) is rigidly connected to a puncturing element drive.

2. Puncturing device according to claim 1, **characterized in that** a visible marker, preferably a colored marker, becomes covered or uncovered when the press-on part (4) is being shifted to the end position.

3. Puncturing device according to claim 2, **characterized in that** the visible marker (17) is disposed on the press-on part (4) and the housing part (7) has a viewing window through which the marker (17) is visible when the press-on part (4) is in the end position.

4. Puncturing device according to any one of the preceding claims, **characterized by**
a securing facility (9) that, in a locking state, locks the triggering means (8) such that no puncturing motion can be triggered, and, in a triggering state, releases the triggering means (8) such that a puncturing motion can be triggered by actuation thereof, whereby
the securing facility (9) is transitioned from the locking state to the triggering state by the testing facility (5,15,20) when the test parameter determined by the testing facility (5,15,20) meets defined minimal requirements, and
a signaling facility (12) for signaling a transition of the securing facility (9) from the locking state to the triggering state and/or for signaling that the securing facility is in the triggering state. Method for preparing a puncturing system for generating a puncturing wound for obtaining a body fluid,

5. Puncturing device according to claim 4, **characterized in that in that** the testing facility (5,15,20) comprises a sensor that can be used to determine, as test parameter, the blood supply status of a body part touching against the press-on part (4).

6. Puncturing device according to any one of the preceding claims, **characterized in that** the pressure sensor (5,15) comprises a snap-action element that snaps from a first configuration to a second configuration when a critical minimal pressure is being applied.

7. Puncturing device according to claim 6, **characterized in that** the snap-action element (15) is a sheet of metal.

8. Puncturing device according to any one of claims 4 to 7, **characterized in that** the testing facility (5,15,20) supports a user in orienting the press-on part (4) with respect to the body part (32) in which a puncturing wound is to be generated by means of signals that convey to the user information regarding the orientation of the press-on part (4) with respect to the body part (32).

9. Puncturing device according to any one of the preceding claims, **characterized in that** the testing facility (5,15,20) facilitates a visual control of the press-on part (4) touching against the body part (32) and/or of the body part (32) touching against the press-on part (4).

10. Puncturing device according to any one of claims 4 to 9, **characterized in that** it comprises a sample reception unit that is used to receive a sample of a body fluid during a collection phase after a puncture, whereby the testing facility (5,15,20) remains active until the end of the collection phase and displays by means of a warning signal any change of the at least one test parameter that is disadvantageous for sample reception.

11. Puncturing device according to claim 10, **characterized in that** it has a measuring unit for examination of a sample of a body fluid thus obtained for a medically significant analyte, whereby the testing facility (5,15,20) is connected to the measuring unit such that an analysis of the sample thus obtained is prevented if a critical change of a test parameter occurs during the collection phase.

## Revendications

1. Appareil de piqûre destiné à réaliser une piqûre pour obtenir un échantillon d'un liquide organique, comprenant
une pièce d'appui (4), destinée à être appliquée contre une partie du corps (32) dans laquelle la piqûre doit être réalisée,
un moyen déclencheur (8), dont l'actionnement permet à un utilisateur, après l'application de ladite pièce d'appui (4), de déclencher un mouvement de piqûre d'un élément de piqûre (3),
un dispositif de contrôle (5, 15, 20), destiné à détecter au moins un paramètre de contrôle duquel dépend une probabilité d'obtention d'échantillon, ledit dispositif de contrôle (5) comprenant un détecteur de pression qui, en tant que paramètre de contrôle, permet de déterminer si la pièce d'appui (4) est soumise à une pression qui dépasse une valeur minimale prédéfinie,
ledit détecteur de pression (5) comprenant un élément élastique contre la force élastique duquel la pièce d'appui (4) peut se déplacer par rapport à une partie de boîtier (7) de telle sorte que lorsqu'une pression est appliquée qui dépasse ladite valeur minimale prédéfinie, la pièce d'appui (4) parvient à une position finale par rapport à ladite partie de boîtier (7), **caractérisé en ce que**
la pièce d'appui (4) est reliée de façon rigide à un entraînement de l'élément de piqûre.

2. Appareil de piqûre selon la revendication 1, **caractérisé en ce que** durant le déplacement de la pièce d'appui (4) vers la position finale, un repère visible, de préférence un repère de couleur, est masqué ou dévoilé.

3. Appareil de piqûre selon l'une des revendications précédentes, **caractérisé en ce que** le repère visible (17) est disposé sur la pièce d'appui (4), et la partie de boîtier (7) comporte une fenêtre de visualisation grâce à laquelle le repère (17) est visible lorsque la pièce d'appui (4) se trouve en position finale.

4. Appareil de piqûre selon l'une des revendications précédentes, **caractérisé par**
un dispositif de sécurité (9), qui en état de blocage bloque le moyen déclencheur (8), empêchant ainsi le déclenchement d'un mouvement de piqûre, et qui en état de déclenchement libère le moyen déclencheur (8), permettant ainsi, par actionnement de ce dernier, de déclencher un mouvement de piqûre,
ledit dispositif de sécurité (9) étant amené par le dispositif de contrôle (5, 15, 20) de l'état de blocage à l'état de déclenchement si le paramètre de contrôle déterminé par le dispositif de contrôle (5, 15, 20) satisfait à des exigences minimales définies, et
un dispositif de signalisation (12) pour signaler un passage du dispositif de sécurité (9) de l'état de blocage à l'état de déclenchement et/ou pour signaler que le dispositif de sécurité se trouve en état de déclenchement.

5. Appareil de piqûre selon la revendication 4, **caractérisé en ce que** le dispositif de contrôle (5, 15, 20) comprend un détecteur qui, en tant que paramètre de contrôle, permet de détecter l'état de la circulation sanguine d'une partie du corps appliquée contre la pièce d'appui (4).

6. Appareil de piqûre selon l'une des revendications précédentes, **caractérisé en ce que** le détecteur de pression (5, 15) comprend un élément à déclic qui sous l'effet d'une pression minimale critique passe d'une première configuration à une deuxième configuration.

7. Appareil de piqûre selon la revendication 6, **caractérisé en ce que** l'élément à déclic (15) est une tôle métallique.

8. Appareil de piqûre selon l'une des revendications 4 à 7, **caractérisé en ce que** le dispositif de contrôle (5, 15, 20) assiste un utilisateur dans l'orientation de la pièce d'appui (4) sur la partie du corps (32) dans laquelle une piqûre doit être réalisée, grâce à des signaux qui communiquent à l'utilisateur une information sur l'orientation de la pièce d'appui (4) par rapport à la partie du corps (32).

9. Appareil de piqûre selon l'une des revendications 4 à 8, **caractérisé en ce que** le dispositif de contrôle (5, 15, 20) permet un contrôle visuel de la pièce d'appui (4) appliquée contre la partie du corps (32) et/ou de la partie du corps (32) appliquée contre la pièce d'appui (4).

10. Appareil de piqûre selon l'une des revendications 4 à 9, **caractérisé en ce qu'**il comprend une unité de recueil d'échantillon permettant après une piqûre, au cours d'une phase de collecte, de recueillir un échantillon de liquide organique, le dispositif de contrôle (5, 15, 20) restant actif jusqu'à la fin de la phase de collecte et indiquant par un signal d'avertissement une variation défavorable pour un recueil d'échantillon dudit au moins un paramètre de contrôle,

11. Appareil de piqûre selon la revendication 10, **caractérisé en ce qu'**il possède un dispositif de mesure pour examiner un analyte médicalement important d'un échantillon de liquide organique obtenu, le dispositif de contrôle (5, 15, 20) étant raccordé audit dispositif de mesure, empêchant ainsi une analyse de l'échantillon obtenu en cas de variation critique d'un paramètre de contrôle survenant au cours de la phase de collecte.
